(19) Europäisches Patentamt
European Patent Office
Office européen des brevets

(11) Publication number: 0 542 220 A1

# (12) EUROPEAN PATENT APPLICATION

(21) Application number: 92119270.4

(22) Date of filing: 11.11.92

(51) Int. Cl.$^5$: C08G 18/32, C08G 18/80, C08G 18/65, C09D 175/04, C09J 175/04, C09K 3/10, C09D 11/00, C07C 271/20, C07C 271/24, C07C 271/28, //(C08G18/32,101:00)

(30) Priority: 12.11.91 US 790896

(43) Date of publication of application: 19.05.93 Bulletin 93/20

(84) Designated Contracting States: AT BE DE DK ES FR GB IT NL SE

(71) Applicant: UNION CARBIDE CHEMICALS & PLASTICS TECHNOLOGY CORPORATION
39 Old Ridgebury Road
Danbury, Connecticut 06817-0001(US)

(72) Inventor: Argyropoulos, John Nicholas
35 Michael Street
Scott Depot, West Virginia 25560(US)
Inventor: Smith, Oliver Wendell
5229 Pioneer Drive
Charleston, West Virginia 25314(US)
Inventor: Koleske, Joseph Victor
1513 Brentwood Road
Charleston, West Virginia 25314(US)

(74) Representative: Brandes, Jürgen, Dr. rer. nat. et al
Wuesthoff & Wuesthoff, Patent- und Rechtsanwälte, Schweigerstrasse 2
W-8000 München 90 (DE)

## (54) Polyurethanes and processes for preparing same.

(57) This invention relates to polyurethanes of various types prepared from certain liquid hydrocarbon diols and/or derivatives of said liquid hydrocarbon diols, and polyfunctional isocyanates. The polyurethanes are useful in preparing coatings, inks, adhesives, sealants, elastomers, and foams. In particular, the invention relates to polyurethanes cured by means of moisture, blocked amines, or related compounds.

EP 0 542 220 A1

## Brief Summary of the Invention

### Technical Field

This invention relates to polyurethanes of various types prepared from certain liquid hydrocarbon diols and/or derivatives of said liquid hydrocarbon diols, and polyfunctional isocyanates. The polyurethanes are useful in preparing coatings, inks, adhesives, sealants, elastomers, and foams. In particular, the invention relates to polyurethanes cured by means of moisture, blocked amines, or related compounds.

### Background of the Invention

Polyurethanes are well known articles of commerce and are marketed in various forms illustrative of which are coatings, sealants, foams, elastomers, and the like. Room temperature curable or cold hardening polyurethanes are known compounds that are commercially sold and used. Such polyurethanes change from the liquid to solid state and harden by reacting and/or crosslinking an isocyanate prepolymer, which is a polymeric material that has free isocyanate groups, with water, usually available as moisture in ambient air. The reaction of water with an isocyanate results in the release of carbon dioxide and the formation of urea groups which may be illustrated as follows:

$$2\ \sim R''\text{-}NCO + H_2O \longrightarrow \sim R''\text{-}\overset{\overset{H}{|}}{N}\text{-}\overset{\overset{O}{\|}}{C}\text{-}\overset{\overset{H}{|}}{N}\text{-}R''\sim$$

wherein R" is a substituted or unsubstituted hydrocarbon residue described in the art and with it understood by those skilled in the art that when multifunctional materials are involved crosslinked products result.

A second method of hardening the coating is to use capped or blocked chain extenders. When the chain extender is heated, the capping or protecting group is cleaved and the chain extender, most often an amine, reacts with the free isocyanate groups and cures the coating. In this latter method the reaction proceeds at a faster rate than in the former method, since the carbon dioxide formation and elimination step is negated and the highly reactive amine is directly formed when the capped molecule is cleaved with moisture.

Other types of polyurethanes include two-package systems in which isocyanate-terminated polyols form adducts and one of the packages and polyols/glycols form the second package of a coating, ink, adhesive or sealant; elastomers which can be used in a molded product or solution coating manner; foams of both a rigid and a flexible nature which are used as cushioning, as insulation, as packaging materials, as oil adsorbants etc.; reaction injection molded plastic parts that are used in the automotive, building, and other industries; and similar products.

Physical characteristics of the polyurethanes and resulting products are affected by the polyol used to make the polyurethane, the polyfunctional isocyanate used, the average functionality of the polyol and the isocyanate, and the ratio of difunctional to trifunctional or higher functionality polyol used in preparing the polyurethane, and the means used to cure or crosslink the polyurethane when such a mechanism is employed. Cure rate can be enhanced by use of small amounts of a catalyst such as dibutyltin dilaurate, stannous octanoate, zinc octanoate and similar organometallic compounds.

The polyurethane products are useful in a variety of ways. Moisture-curable polyurethanes are used in varnishes of various types, including those used in finishing woodwork and doors in homes and industrial building, athletic surfaces, and in similar ways. Illustrative of other end uses for various polyurethane products include coatings for concrete, fabrics, golf balls, seamless flooring, and leather; cast and molded products; elastomers; fibers; foams; and the like.

Although the known moisture curing polyurethanes have many uses, they also have shortcomings among which are moisture and chemical resistance. Such attack can lead to failure in a functional and/or a decorative sense. An improvement in these properties is highly desirable since it would result in longer usable lifetimes of the coated material, savings in energy and labor resources by decreasing the need for repair, and with fewer times of repair be ecologically sound because less total coating would be required over the lifetime of use and it would increase the time before replacement is required.

Moisture-curable polyurethanes are well-known articles of commerce and are described in ASTM D 16 as Type II, one-package moisture cured products that are urethane coatings characterized as having free isocyanate groups and capable of conversion to useful films by reaction of the free isocyanate groups with adventitious or other water usually in the form of moisture. Also included in the purvey of this invention are those polyurethanes that are described in ASTM D 16 as Type III, one-package heat cured products that are urethane coatings that dry or cure by thermal release of blocking agents which results in the regeneration of active isocyanate groups that afterward cure with substance containing active hydrogen groups including adventitious or other water. Other useful polyurethanes include two-package catalyzed polyurethanes (ASTM Type IV) wherein one package contains a prepolymer or adduct having free isocyanate groups capable of forming useful films by combining with a relatively small quantity of catalyst, accelerator, or crosslinking agent such as a polyol or polyamine contained in a second package; two-package polyol polyurethanes (ASTM Type V) which comprise systems wherein one package contains a prepolymer or adduct or other polyisocyanate capable of forming useful films by combining with a substantial quantity of a second package containing a resin having active hydrogen groups with or without use of a catalyst; and one-package nonreactive lacquer polyurethanes (ASTM Type VI) characterized by the absence of any significant quantity of free isocyanate or other functional groups and convert to solids primarily by solvent evaporation.

Disclosure of the Invention

It has been found that polyurethanes, particularly moisture-curable polyurethanes, can be prepared from certain liquid hydrocarbon diols and/or derivatives of said liquid hydrocarbon diols, polyfunctional isocyanates, and optionally other polyols. The liquid hydrocarbon diols are comprised of primary hydroxyl groups and 8 or more carbon atoms in which the primary hydroxyl groups are separated by 4 or more carbon atoms linearly arranged and in which at least one of said carbon atoms linearly arranged is a disubstituted carbon atom or at least 2 of said carbon atoms linearly arranged are monosubstituted carbon atoms, said liquid hydrocarbon diol existing as a liquid at a temperature of 35°C or less.

This invention also relates to a process for preparing polyurethanes which comprises contacting a liquid hydrocarbon diol comprised of primary hydroxyl groups and 8 or more carbon atoms in which the primary hydroxyl groups are separated by 4 or more carbon atoms linearly arranged and in which at least one of said carbon atoms linearly arranged is a disubstituted carbon atom or at least 2 of said carbon atoms linearly arranged are monosubstituted carbon atoms, said liquid hydrocarbon diol existing as a liquid at a temperature of 35°C or less, and/or a derivative of said liquid hydrocarbon diol, with a polyfunctional isocyanate and optionally other polyols to form said polyurethane.

The preferred polyurethanes are comprised of isocyanate-terminated species that are of the general form given by Formula I below when prepared from two moles of diisocyanate and one mole of a particularly useful class of diols:

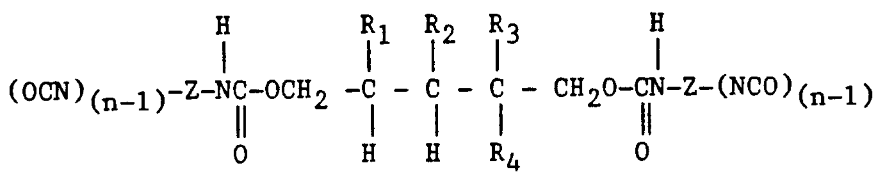 (I)

wherein $R_1$ is hydrogen or linear or branched alkyl having from 1 to 3 carbon atoms, $R_2$, $R_3$ and $R_4$ are the same or different and are hydrogen or linear or branched alkyl having from 1 to 4 carbon atoms, Z is the same or different and is a substituted or unsubstituted hydrocarbon residue, and n is a value of from about 2 to about 6.

Polyurethanes of this type are useful in a variety of ways illustrative of which are moisture-curable polyurethanes that are useful as coatings, inks, sealants, and adhesives; prepolymers that may be reacted with polyols, polyamines or polyimines that can be made into relatively high or high molecular polyurethanes or polyurethane/ureas that are useful as elastomers, foams, fibers, molded parts from reaction injection molding and other techniques; alcohols, amines, imines, alkanolamines, to produce a variety of products useful in their own right as chemical intermediates, solvents, medical implants, pharmaceuticals, pharmaceutical intermediates; herbicides, biocides, dye intermediates, stabilizers, antioxidants, fungicides for photographic gelatins, textile chemicals, oil absorbents and the like.

Detailed Description

It has been discovered that polyurethanes, particularly moisture-curable polyurethanes (ASTM Type II) and thermally-curable blocked polyurethanes (ASTM Type III), can be prepared by reacting liquid hydrocarbon diols comprised of primary hydroxyl groups and 8 or more carbon atoms in which the primary hydroxyl groups are separated by 4 or more carbon atoms linearly arranged and in which at least one of said carbon atoms linearly arranged is a disubstituted carbon atom or at least 2 of said carbon atoms linearly arranged are monosubstituted carbon atoms, said liquid hydrocarbon diol existing as a liquid at a temperature of 35 °C or less, and/or derivatives of said liquid hydrocarbon diols, multifunctional isocyanates, and optionally other polyols to form isocyanate-terminated products that are useful as intermediates that can be cured by means of adventitious water usually supplied in the form of atmospheric moisture, of supplied moisture, by thermal means, or by a combination of thermal means and moisture obtained in an adventitious or supplied manner.

Other polyurethanes that fall within the purvey of this invention include the ASTM Type IV two-package catalyzed polyurethanes wherein one package contains a prepolymer or adduct having free isocyanate groups capable of forming useful films by combining with a relatively small quantity of catalyst, accelerator, or crosslinking agent such as a polyol or polyamine contained in a second package; ASTM Type V two-package polyol polyurethanes which comprise systems wherein one package contains a prepolymer or adduct or other polyisocyanate capable of forming useful films by combining with a substantial quantity of a second package containing a resin having active hydrogen groups with or without use of a catalyst; and ASTM Type VI one-package nonreactive lacquer polyurethanes characterized by the absence of any significant quantity of free isocyanate or other functional groups and convert to solids primarily by solvent evaporation.

The preferred liquid hydrocarbon diols for use in this invention can be represented by the formula:

$$HO - R' - OH$$

wherein R' is a substituted hydrocarbon residue having 8 or more carbon atoms in which the primary hydroxyl groups are separated by 4 or more carbon atoms linearly arranged and in which at least one of said carbon atoms linearly arranged is a disubstituted carbon atom or at least 2 of said carbon atoms linearly arranged are monosubstituted carbon atoms, said liquid hydrocarbon diol existing as a liquid at a temperature of 35 °C or less.

Other preferred liquid hydrocarbon diols useful for preparation of the polyurethanes of this invention are substituted 1,5-pentanediols of Formula II:

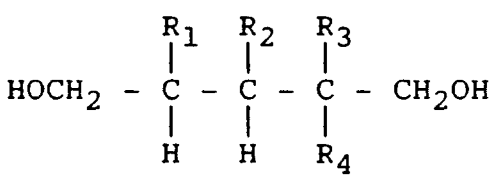

(II)

wherein $R_1$ is hydrogen or linear or branched alkyl having 1 to 3 carbon atoms, and $R_2$, $R_3$, and $R_4$ are the same or different and are hydrogen or linear or branched alkyl having 1 to 4 carbon atoms, said liquid hydrocarbon diol existing as a liquid at a temperature of 35 °C or less; provided (a) at least 2 of $R_1$, $R_2$, $R_3$ and $R_4$ are other than hydrogen; (b) the total number of carbon atoms in $R_1$, $R_2$, $R_3$ and $R_4$ together is 5 or more except as follows: (i) when $R_2$ is ethyl and one of $R_1$, $R_3$ and $R_4$ is methyl, then the remaining 2 of $R_1$, $R_3$ and $R_4$ can be hydrogen; (ii) when $R_3$ and $R_4$ are independently methyl and propyl, then $R_1$ and $R_2$ can be hydrogen; and (iii) when $R_1$ is methyl and $R_2$ is ethyl and one of $R_3$ and $R_4$ is methyl, then the remaining $R_3$ or $R_4$ can be hydrogen; (c) when the total number of carbon atoms in $R_1$ and $R_2$ is 4 or more, then the total number of carbon atoms in $R_2$ is 3 or less except when $R_1$ is propyl and $R_3$ and $R_4$ are hydrogen, then $R_2$ can be butyl; and (d) when $R_1$ is ethyl and $R_2$ is propyl, then $R_3$ is other than ethyl.

The liquid hydrocarbon diols useful in this invention, for example, the substituted liquid 1,5-pentanediols represented by Formula II above, can be prepared by a process comprising:

(a) contacting a substituted vinyl ether with a substituted or unsubstituted acrolein to form a substituted 3,4-dihydropyran;

(b) contacting the substituted 3,4 – dihydropyran with an acid catalyst to form a substituted dialdehyde; and

(c) hydrogenating the substituted dialdehyde in the presence of a catalyst to form a liquid hydrocarbon diol represented by Formula II above.

More particularly, the liquid, substituted 1,5 – pentanediols represented by Formula II above can be prepared by reacting acrolein or substituted acrolein with a substituted vinyl ether as follows:

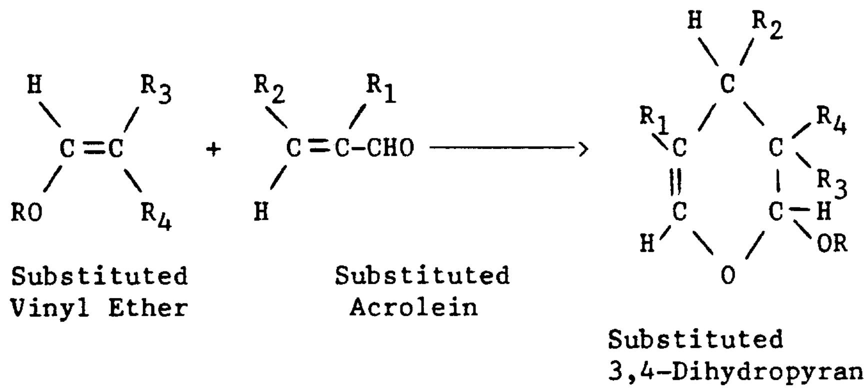

Substituted Vinyl Ether

Substituted Acrolein

Substituted 3,4-Dihydropyran

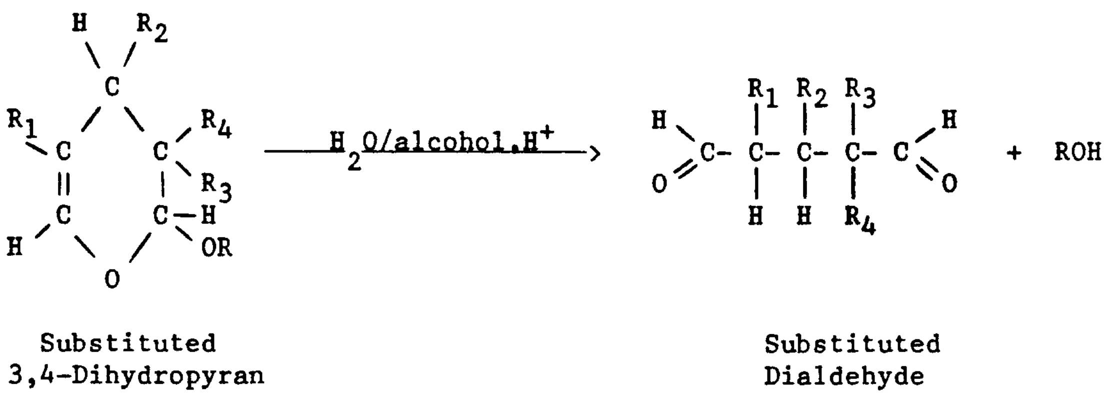


Substituted 3,4-Dihydropyran

Substituted Dialdehyde

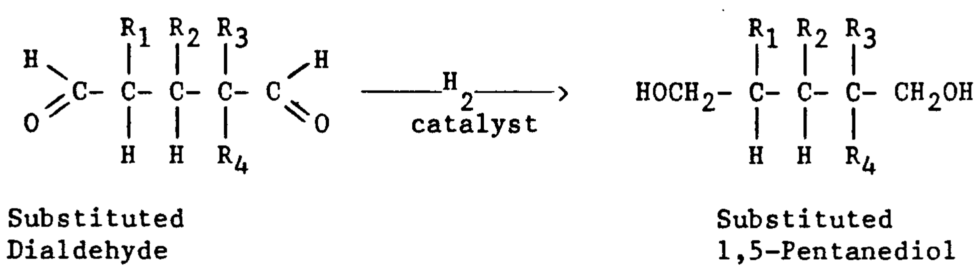


Substituted Dialdehyde

Substituted 1,5-Pentanediol

wherein $R_1$, $R_2$, $R_3$ and $R_4$ are as defined above and R is a substituted or unsubstituted hydrocarbon residue, preferably a linear or branched alkyl having from 1 to about 8 carbon atoms.

Illustrative of suitable substituted vinyl ethers useful in preparing the liquid hydrocarbon diols include, among others, alkyl vinyl ethers such as methyl vinyl ether; methyl (2 – methyl vinyl) ether which has the structure:

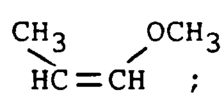

;

methyl (2-ethyl vinyl) ether, which has the structure:

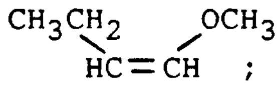
;

methyl (2,2-dimethyl vinyl) ether; methyl (2-methyl, 2-propyl vinyl) ether; methyl (2-butyl, 2-methyl vinyl) ether; ethyl vinyl ether; ethyl (2-methyl vinyl); ethyl (2-ethyl vinyl) ether; ethyl (2,2-dimethyl vinyl) ether; ethyl (2-methyl, 2-propyl vinyl) ether; ethyl (2-butyl, 2-methyl vinyl) ether; n-propyl and i-propyl vinyl ethers; butyl vinyl ethers such as n-butyl vinyl ether, s-butyl vinyl ether, i-butyl vinyl ether, and t-butyl vinyl ether; amyl vinyl ethers, and the like; divinyl ethers such as triethylene glycol divinyl ether, 1,4-cyclohexane dimethanol divinyl ether, trivinyl ethers, and the like. It is preferred that alkyl vinyl ethers with up to 3 carbon alkyl groups and one- to three-carbon alkyl (alkyl vinyl) ethers with alkyl vinyl groups of up to 8 carbon atoms are used.

Illustrative of suitable acroleins useful in preparing the liquid hydrocarbon diols include, among others, acrolein; 2-ethyl-2-butenal; 2-methyl-2-butenal; 2-(n-propyl)-2-butenal; 2-(i-propyl)-2-butenal; 2-methyl-2-pentenal, which has the structure:

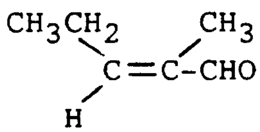
;

2-ethyl-2-pentenal; 2-(n-propyl)-2-pentenal; 2-(i-propyl)-2-pentenal; 2-(n-butyl)-pentenal; 2-(i-butyl)-pentenal; 2-(s-butyl)-pentenal; 2-(t-butyl)-pentenal; 2-amyl pentenals; 2-ethyl-2-hexenal, which has the structure:

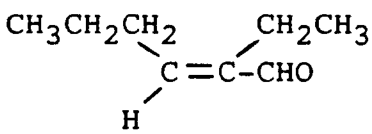
;

2-methyl-2-hexenals; 2-(n-propyl)-2-hexenals; 2-(i-propyl)-2-hexenals; 2-(n-butyl)-2-hexenals; 2-(i-butyl)-2-hexenals; 2-(s-butyl)-2-hexenals; 2-(t-butyl)-2-hexenals; 2-amyl hexenals; and the like.

Illustrative of suitable substituted 3,4-dihydropyrans prepared in reaction step (a) above include, for example, 2-alkoxy-5-ethyl-4-propyl-3,4-dihydro-1,2-pyran, 2-alkoxy-4-ethyl-5-methyl-3,4-dihydro-1,2-pyran, 2-alkoxy-4-ethyl-3,5-dimethyl-3,4-dihydro-1,2-pyran, 2-alkoxy-5-ethyl-3-methyl-4-propyl-3,4-dihydro-1,2-pyran, 2-alkoxy-3,4-diethyl-5-methyl-3,4-dihydro-1,2-pyran, 2-alkoxy-4-ethyl-3,3',5-trimethyl-3,4-dihydro-1,2-pyran, 2-alkoxy-3,3'-dimethyl-4-propyl-3,4-dihydro-1,2-pyran, 2-alkoxy-3-methyl-3'-propyl-3,4-dihydro-1,2-pyran, 2-alkoxy-4-ethyl-5-methyl-3-methyl-3'-propyl-3,4-dihydro-1,2-pyran, 2-alkoxy-5-ethyl-3-methyl-3',4-dipropyl-3,4-dihydro-1,2-pyran, 2-alkoxy-3-butyl-3'-ethyl-3,4-dihydro-1,2-pyran, 2-alkoxy-3-butyl-3',4-diethyl-5-methyl-3,4-dihydro-1,2-pyran, 2-alkoxy-3-butyl-3',5-diethyl-4-propyl-3,4-dihydro-1,2-pyran, and the like. For purposes of these illustrative substituted 3,4-dihydropyrans, alkoxy refers to methoxy, ethoxy, n-propoxy, isopropoxy, n-butoxy, i-butoxy, s-butoxy, t-butoxy, and the like.

The step (a) reaction can be conducted at a temperature of from about 160 °C to 280 °C for a period of about 1 hour to about 7 days with the longer time being used at the lower temperature, preferably from about 180 °C to about 270 °C for about 1 hour to about 5 days, and more preferably at about 200 °C to 260 °C for about 1 hour to about 48 hours. During the reaction, from less than 0.01 percent by weight to about 5 percent by weight of the total weight of the starting materials, preferably from about 0.01 percent by weight to about 2 percent by weight, of a free radical inhibitor can be added to the reaction mass. Illustrative of such free radical inhibitors are 2,6-ditertiarybutyl-4-methyl phenol, hydroquinone,

hydroquinone monomethyl ether, and the like. A particularly useful inhibitor is hydroquinone.

The step (a) reaction can be conducted over a wide range of pressures ranging from atmospheric pressure to superatmospheric pressures, e.g., from about 1 atmosphere to about 100 atmospheres or greater. It is preferable to conduct the step (a) reaction at pressures of from about atmsopheric to about 75 atmospheres. The step (a) reaction is preferably effected in the liquid or vapor states or mixtures thereof.

The molar ratio of substituted vinyl ether to acrolein compound in the step (a) reaction is not narrowly critical and can range from about 0.05:1 or less to about 50:1 or greater, preferably from about 0.1:1 to about 10:1.

Illustrative of acid catalysts suitable for ring opening of the substituted 3,4-dihydropyran intermediates to form substituted dialdehydes in accordance with reaction step (b) above are mineral acids, including sulfuric acid, hydrochloric acid, phosphoric acid, triflic acid and its salts, sulfonic acids; organic acids including acetic acid, chloroacetic acid, oxalic acid; crosslinked acidic resins such as the various ion exchange resins including Amberlite® CG-400, Amberlite® IR-118; Amberlite® IR120(plus), Dowex® MSC-1, Dowex® M-31, Dowex® M32, Dowex® 50X2-100, Dowex® 50X2-200, Dowex® 50X2-400, Dowex® 50X4-400, Dowex® 50X8-100, Dowex® 50X8-200, Dowex® 50X8-400, Nafion® 117, Nafion® 417, Nafion® NR50, Nafion® perfluorinated powder, similar crosslinked acidic resins; perfluorinated polymers which contain sulfonic acid groups such as XUS-40036.02 (Dow Chemical Company); and the like. The illustrative ion exchange resins above, as well as others, are available from Aldrich Chemical Company, Inc.

The acid catalysts employed in the ring-opening of the substituted 3,4-dihydropyran intermediates are preferably used in conjunction with water; alcohols such as methanol, ethanol, isopropanol, n-propanol, n-butanol, sec-butanol, isobutanol, and tert-butanol, amyl alcohols as well as higher alcohols; glycol ethers such as ethoxy ethanol, methoxy ethanol, 1-methoxypropane, methoxyethoxy ethanol; glyme; and the like, as well as mixtures of water and other solvents. The amount of acid catalyst used in reaction step (b) is dependent on the particular catalyst employed and can range from about 0.01 weight percent or less to about 10 weight percent or greater of the total weight of the starting materials.

The acid dialdehyde reaction mass can be washed with water and/or aqueous solution of neutralizing agents. Illustrative of such neutralizing agents are sodium acetate, potassium acetate, sodium bicarbonate, sodium carbonate, potassium bicarbonate, potassium carbonate, and the like.

Illustrative of suitable substituted dialdehydes prepared in reaction step (b) above include, for example, 3-ethyl-2-methyl-1,5-pentanedial, 2-ethyl-3-propyl-1,5-pentanedial, 3-ethyl-2,4-dimethyl-1,5-pentanedial, 2-ethyl-4-methyl-3-propyl-1,5-pentanedial, 3,4-diethyl-2-methyl-1,5-pentanedial, 3-ethyl-2,4,4'-trimethyl-1,5-pentanedial, 2-ethyl-4,4'-dimethyl-3-propyl-1,5-pentanedial, 2-methyl-2'-propyl-1,5-pentanedial, 3-ethyl-2,4-dimethyl-4'-propyl-1,5-pentanedial, 2-ethyl-4-methyl-3,4'-dipropyl-1,5-pentanedial, 2-butyl-2'-ethyl-1,5-pentanedial, 4-butyl-3,4-diethyl-2-methyl-1,5-pentanedial, 4-butyl-2,4'-diethyl-3-propyl-1,5-pentanedial, and the like.

The step (b) reaction can be conducted over a wide range of pressures ranging from atmospheric or subatmospheric pressures to superatmospheric pressures, e.g., from about 1 atmosphere or less to about 25 atmospheres or greater. It is preferable to conduct the step (b) reaction at pressures of from about 1 atmosphere to about 10 atmospheres. The step (b) reaction is preferably effected in the liquid or vapor states or mixtures thereof.

The temperature of the step (b) reaction may be as low as about ambient temperature to about 300° C. Preferably, the reaction temperature ranges from about 50° C to about 200° C, and more preferably from about 60° C to about 120° C.

Illustrative of catalysts useful in the hydrogenation step (c) involving reduction of substituted dialdehydes include, for example, Raney-type compounds such as Raney nickel and modified Raney nickels; molybdenum-promoted nickel, chromium-promoted nickel, cobalt-promoted nickel; platinum; palladium; iron; cobalt molybdate on alumina; copper chromite; barium promoted copper chromite; tin-copper couple; zinc-copper couple; aluminum-cobalt; aluminum-copper; and aluminum-nickel; platinum; nickel; and the like. The amount of hydrogenation catalyst used in step (c) is dependent on the particular catalyst employed and can range from about 0.01 weight percent or less to about 10 weight percent or greater of the total weight of the starting materials.

The particular reaction conditions for the step (c) hydrogenation reaction are not narrowly critical, and can be any effective hydrogenation procedures sufficient to produce the substituted 1,5-pentanediols of this invention. The step (c) reaction can be carried out at temperatures of from about ambient temperature to about 250° C, preferably from about 70° C to about 200° C, and more preferably from 90° C to 150° C. The step (c) reaction preferably may be conducted at pressures of from about 5 atmospheres to about 100

atmospheres, and more preferably from about 10 atmospheres to about 75 atmospheres.

Illustrative of suitable substituted 1,5-pentanediols useful in preparing the polyurethanes of this invention include, for example, 3-ethyl-2-methyl-1,5-pentanediol, 2-ethyl-3-propyl-1,5-pentanediol, 2,4-dimethyl-3-ethyl-1,5-pentanediol, 2-ethyl-4-methyl-3-propyl-1,5-pentanediol, 2,3-diethyl-4-methyl-1,5-pentanediol, 3-ethyl-2,2,4-trimethyl-1,5-pentanediol, 2,2-dimethyl-4-ethyl-3-propyl-1,5-pentanediol, 2-methyl-2-propyl-1,5-pentanediol, 2,4-dimethyl-3-ethyl-2-propyl-1,5 pentanediol, 2,3-dipropyl-4-ethyl-2-methyl-1,5-pentanediol, 2-butyl-2-ethyl-1,5-pentanediol, 2-butyl-2,3-diethyl-4-methyl-1,5-pentanediol, 2-butyl-2,4-diethyl-3-propyl-1,5-pentanediol, 3-butyl-2-propyl-1,5-pentanediol and the like, including mixtures thereof.

Illustrative derivatives based on the liquid hydrocarbon diols that are useful in preparing the polyurethanes of this invention include, for example, polyesters, silicone-containing compounds, polyols initiated with said diols, and the like, including mixtures thereof. This invention is not intended to be limited in any manner by the permissible derivatives of liquid hydrocarbon diols.

The polyester derivative products useful in this invention include, for example, those represented by the structural formula:

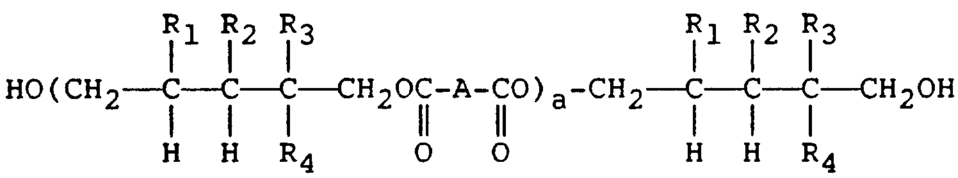

(III)

wherein $R_1$ is the same or different and is hydrogen or linear or branched alkyl having from 1 to 3 carbon atoms, $R_2$, $R_3$ and $R_4$ are the same or different and are hydrogen or linear or branched alkyl having from 1 to 4 carbon atoms, A is the same or different and is a substituted or unsubstituted hydrocarbon residue, preferably aryl such as phenylene, substituted phenylene, alkyl, cyclohexyl, substituted cyclohexyl and the like, and a is a value from about 1 to about 300 or greater, preferably from 1 to about 150, and more preferably from about 1 to about 75 or less.

Optionally, up to about 60 weight percent, preferably up to about 40 weight percent of other di-, tri-, tetra-, and higher-functionality polyols may be used in combination with the liquid hydrocarbon diols of Formula II to form the polyesters of Formula III. Suitable polyols include, for example, ethylene glycol, diethylene glycol, propylene glycol, dipropylene glycol, polyoxypropylene diols and triols, neopentyl glycol, esterdiols such as Esterdiol-204 and ethoxylated and propoxylated esterdiols, ethylene oxide/propylene oxide copolymer polyols, polyether polyols, polycarbonate polyols, poly(alkylene oxide) polyols, 1,3-propanediol, 1,4-butanediols, poly(tetramethylene oxide) polyols, 1,5-pentanediols other than those of Formula II, 1,6-hexanediols, 2-ethyl-1,3-hexanediol, 1,7-heptanediol, and higher linear and branched hydrocarbon diols, polylactone diols and triols such as the poly-$\epsilon$-caprolactone polyols; halogenated diols such as 3-chloro-1,2-propanediol, 2,3-dibromo-1,4-butanediol; triols and higher hydroxyl-functional polyols such as trimethylolpropane, pentaerythritol, dipentaerythritol, sorbitol, sucrose; hydroquinone and substituted hydroquinones, bisphenols such as Bisphenol A, Bisphenol C, Bisphenol F, as well as others; 1,2-cyclohexanediols, 1,3-cyclohexanediols, 1,4-cyclohexanediols, 1,4-cyclohexane dimethanol, xylenediols, 2,2,4,4-tetramethyl-1,3-cyclobutanediol, and the like, including mixtures thereof.

Illustrative of the polyfunctional carboxylic acids that can be used to prepare the polyester derivative products useful in this invention include, for example, oxalic acid, malonic acid, succinic acid, glutaric acid, adipic acid, pimelic acid, suberic acid, azelaic acid, sebacic acid, undecanedioic acid, dodecanedioic acid, tridecanedioic acid, maleic acid, fumaric acid, 2-methyl-cis-2-butenedioic acid, 2-methylenesuccinic acid, 1,1-cyclobutanedicarboxylic acid, norcamphoric acid, tetrahydrophthalic acid, methyl-tetrahydrophthalic acid, 1,1-cyclohexanedicarboxylic acid, hexahydrophthalic acid, 1,4-cyclohexanedicarboxylic acid, chlorendic acid, 1,4-benzenediacetic acid, phthalic acid, isophthalic acid, trimellitic acid, any other polyfunctional carboxylic acid including those having substituents thereon such as alkyl or alkoxy groups, nitro, halogen, aryl, carboxyl or any other group that will not unduly interfere with the reaction and the like as well as mixtures of such acids and mixtures of such acids with acid anhydrides.

Illustrative of the acid anhydrides that can be used to prepare the polyester derivative products useful in this invention include, for example, trimellitic anhydride, tetrahydrophthalic anhydride, phthalic anhydride,

isophthalic anhydride, benzophenone dicarboxylic acid anhydride, succinic anhydride, glutaric anhydride, napthoic anhydride, clorendic anhydride, itaconic anhydride, maleic anhydride, or any other intramolecular anhydride including those having substituents thereon such as alkyl or alkoxy groups, nitro, halogen, aryl, carboxyl or any other group that will not unduly interfere with the reaction and the like as well as mixtures of anhydrides or mixtures of anhydrides and polyfunctional carboxylic acids.

The polyester derivatives can be prepared by heating conventional amounts of the liquid hydrocarbon diols, optional polyols, polyfunctional carboxylic acids and/or acid anhydrides at an elevated temperature and removing water of condensation. The process of condensation is rate enhanced if catalysts are used. The catalysts that may be used to prepare the polyesters useful in the compositions of this invention are those known to persons skilled in the art of polyester preparation, illustrative of which are dibutyltin oxide, antimony oxide, tin oxide, titanium alkoxides, alkali metal salts or metallic salts of manganese, cadmium, magnesium, zinc, cobalt, tin, and the like.

The silicone-containing derivative compounds useful in this invention can be prepared by conventional methods by either end capping, coupling, or other reaction when Formula II liquid hydrocarbon diols or mixtures of Formula II liquid hydrocarbon diols and optionally other polyols are reacted with silanes. Illustrative of the silane-containing derivative compounds include, for example, the following:

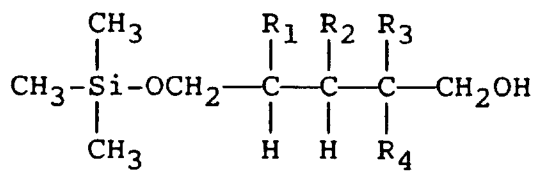

when 1 mole of trimethylchlorosilane and 1 mole of Formula II diol are reacted,

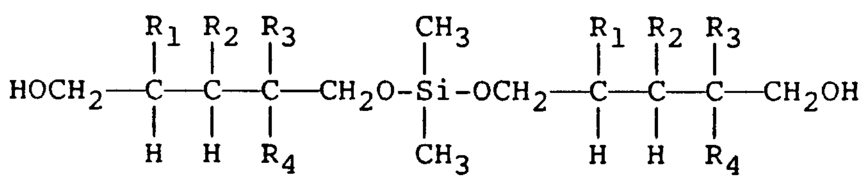

when one mole of dimethyldichlorosilane and two moles of Formula II diol are reacted,

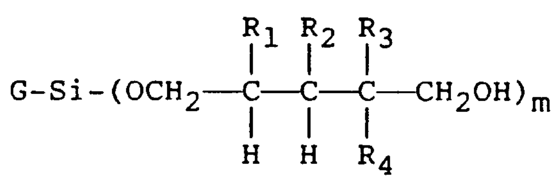

wherein $R_1$, $R_2$, $R_3$ and $R_4$ are as defined above, m is 3 or 4 and G is methyl when m is 3 and nonexistent when m is 4 when 3 moles (m = 3) or 4 moles (m = 4) of Formula II diol are reacted with methyltrichlorosilane or tetrachlorosilane respectively, and the like. It is preferred that the silane-containing product have residual hydroxyl groups. It is understood by those skilled in the art that when polyfunctional compounds are combined, a variety of products, including chain extended products, can be obtained. Illustrative of the silanes that can be used to produce the silane-containing compounds useful in the compositions of the invention include, for example, chloroalkylchloro and arylchlorosilanes, diphenylethylchlorosilane, trimethylchlorosilane, dimethyldichloromethylsilane, triphenylchlorosilane, methyldichlorosilane, dimethylethylchlorosilane, dichlorosilane; alkoxysilanes such as methoxysilane, dimethoxysilane, diethyoxysilane, triethoxysilane, dimethylmethoxychlorsilane, dimethylmethoxysilane, tris(methoxy)-3-chloropropylsilane, and the like, including mixtures thereof.

Illustrative polyols that can be used in combination with the Formula II liquid hydrocarbon diols in preparation of the silicone-containing derivative products useful in this invention include, for example, diethylene glycol, 1,4-butanediol, 1,6-hexanediol, 1,4-dihydroxyquinone, 2,2-dimethyl-1,3-propanediol, hydroxyl-terminated polyesters, ethylene oxide/propylene oxide copolymer polyols, poly(ethylene oxide) polyols, poly(alkylene oxide) polyols, polyether polyols, poly(tetramethylene oxide) polyols, polycarbonate polyols, polylactone polyols, and the like, including mixtures thereof.

The polyol derivative products formed by ring-opening polymerization and initiated with the liquid hydrocarbon diols of Formula II and useful in this invention include, for example, polyols formed by reaction of one mole of the Formula II compound and from about one to about 10 moles of $\epsilon$-caprolactone, substituted $\epsilon$-caprolactone, $\delta$-valerolactone, substituted $\delta$-valerolactones, or a mixture of such lactones or other copolymerizable lactones; or from 1 to about 10 moles of propylene oxide, epichlorohydrin, 1,2-butylene oxide, or ethylene oxide or mixtures of such oxides, with it preferred that from 1 to about 4 moles of ethylene oxide by used alone or in the mixtures. If desired both alkylene oxide and lactone units can be present in the polyols. Methods of preparation of such polyols are well known to those skilled in the art of lactone or alkylene oxide polymerization.

The preferred isocyanate-terminated polyurethanes of this invention can be depicted as follows when a Formula II diol is reacted with a polyfunctional isocyanate represented by the structure:

$Z-(NCO)_n$

to form certain isocyanate-terminated polymers of the general formula:

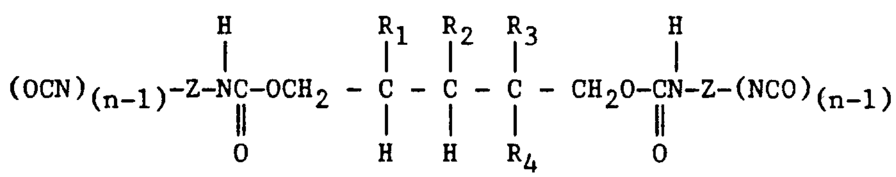

wherein $R_1$, $R_2$, $R_3$ and $R_4$ are as defined above, Z is the same or different and is a substituted or unsubstituted hydrocarbon residue, preferably aryl such as phenylene, substituted phenylene, alkyl, cyclohexyl, substituted cyclohexyl and the like, and n is a value of from about 2 to about 6, preferably from 2 to about 3.

The amount of liquid hydrocarbon diol and polyol to be used for preparation of the polyurethanes of this invention is not narrowly critical; however, it is preferably equal to the number of equivalents of isocyanate minus one so that an essentially isocyanate-capped polyurethane as depicted in Formula I is obtained.

It is understood by those skilled in the art that higher molecular weight species can form by chain extension during the reaction in which the liquid hydrocarbon diol is capped with the multifunctional isocyanate, and that when n is greater than 2, branched structures of higher molecular weight can be formed. A wide variety of modifying polyols can be added to the Formula II compounds when the polyurethanes are prepared or said polyols can be used as the one of the packages in two-package polyurethanes which contain Formula I products as one of the packages. In a specific embodiment of this invention, monoisocyanates can be used to partially cap the liquid hydrocarbon diols of the invention and impart special properties such as flame resistance when halogenated aryl monoisocyanates are used.

Illustrative of the polyfunctional isocyanates that can be used to prepare the polyurethanes of this invention include, for example, 2,4-toluene diisocyanate and 2,6-toluene diisocyanate as well as mixtures of 2,4- and 2,6-toluene diisocyanate; 4,4'-diphenylmethane diisocyanate (MDI), 4,4'-dicyclohexyldiisocyanate or reduced MDI, meta- and para-tetramethyl xylene diisocyanate, 3-isocyanatomethyl-3,5,5-trimethylcyclohexyl-isocyanate, hexamethylene diisocyanate, 2,2,4- and 2,4,4-trimethylenehexamethylene diisocyanate, meta-and para-phenylene diisocyanate, isophorone diisocyanate; 2,4-, 2-6-and 2,4-/2,6-bromotoluene diisocyanate, 4-bromo-meta-phenylene diisocyanate, ortho- and meta-trifluoromethylphenylisocyanate; ortho, meta-, and para-fluorophenylisocyanate; 4,6-dibromo-meta-phenylene diisocyanate; ortho-, meta-, and para-chlorophenyl isocyanate, 4,4',4''-triisocyanatotriphenylmethane, and the like, including mixtures thereof.

Other polyols can be used in combination with the particular Formula II 1,5-pentanediols when the Formula I polyurethanes and other polyurethanes of this invention are prepared. The polyols contain two or more free hydroxyl groups, preferably from about two to about eight, and most preferably from two to about three free hydroxyl groups. Of particular interest are Type II or Type III polyurethanes that contain minor amounts of from about less than one percent by weight to about 45 percent by weight, preferably from about one to about 25 weight percent, and most preferably from about one to about 15 weight percent of a

tri – or higher hydroxyl functionality polyol.

Illustrative of the polyols useful for addition to the Formula II 1,5 – pentanediols to form the polyurethanes of this invention include, for example, ethylene glycol, diethylene glycol, 1,4 – butanediol, 1,6 – hexanediol, 2,2,4 – trimethyl – 1,5 – pentanediol, Esterdiol 204, ethoxylated and propoxylated Esterdiol 204, trimethylolpropane, ethoxylated and propoxylated trimethylolpropane, 1,4 – cyclohexane dimethanol, 1,4 – phenylene dimethanol; the various caprolactone polyols including di – and trifunctional caprolactone polyols such as TONE® – 0200, – 0210, – 0230, – 0240, – 0260, – 0301, – 0305, and – 0310 which are commercially available from Union Carbide Chemicals and Plastics Company Inc.; propylene glycol and poly(propylene oxide) polyols, poly(tetramethylene oxide) polyols, polyester polyols, polyether polyols, poly(alkylene oxide) polyols, polyoxyethylene polyols, polycarbonate polyols, and the like, including mixtures thereof.

Catalysts that can be used to catalyze the isocyanate/hydroxyl reaction that is involved in urethane linkage formation are known to those skilled in the art. Illustrative of such catalysts which can be used in conventional amounts include organometallics such as stannous octanoate, zinc octanoate, dibutyltin dilaurate; amines such as the alkanolamines, tetramethyldiamines; and the like.

Catalysts that can be used to promote the moisture cure of polyurethanes are known to those skilled in the art. Illustrative of such catalysts are amines, alkanol amines, and organometallics such as methyl diethanolamine, cobalt naphthenate, lead naphthenate, dibutyltin dilaurate, and the like.

Blocked compounds that can be used to promote curing of polyurethanes include Schiff bases as described by G. Schleier and F. Gerold in Proceedings of Polyurethanes World Congress 1987 held in Aachen, Germany on 09/29 – 02/87, pages 323 – 327, phenol – blocked isocyanates including phenol derivatives of the Formula I compounds, blocked amines, and the like. When such blocked compounds are employed, it is necessary to heat the coating system to a temperature such that unblocking will occur which can be from about 100 ° C to about 200 ° C. If only blocked isocyanates are involved, it is necessary to cool the deblocked systems to room temperature and allow moisture to cause curing. If polyols are added or if blocked amines are employed, curing will take place in the absence of moisture at the elevated temperature.

When two – package polyurethanes are made from the Formula I isocyanate – terminated compounds of this invention, the optional modifying polyols can also be used individually or in admixture as one of the two – packages.

Thermoplastic polyurethanes that can be used for molding shaped articles, elastomers, fibers, or in solution form as coatings can be prepared from diisocyanate terminated Formula I compounds of this invention, as illustrated by the following equation in which a Formula II diol was used and n is equal to 2:

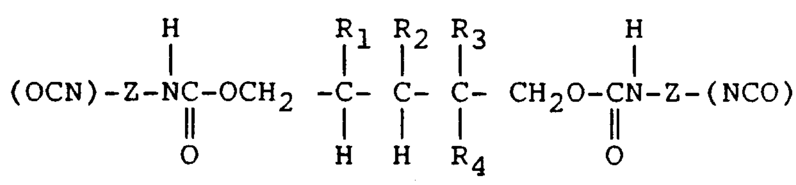

and a polyol which may be represented as:

HO – (POLYOL) – OH

and the resulting polyurethane represented by the formula:

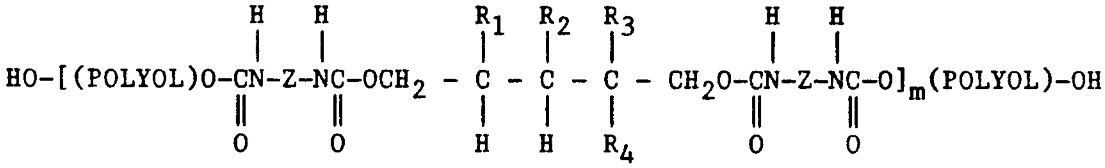

wherein $R_1$, $R_2$, $R_3$, $R_4$ and Z are as described above, POLYOL is a substituted or unsubstituted hydrocarbon residue, and m has a value of from about 25 or less to about 150 or greater with it understood by those skilled in the art that the molecular weight of the thermoplastic polyurethane is dependent on the

particular diisocyanate and difunctional polyol ingredients used. If desired, small amounts of trifunctional or higher functionality isocyanates and/or polyols can be used to alter toughness and other properties. The amount of these higher functionality isocyanates that can be used is dependent on the molecular weight of the various compounds used and is such that insolubilization does not take place. It is usually preferable to prepare thermoplastic polyurethanes that are to be used for coatings and inks in an inert solvent such as those described below. The polyurethanes may also be prepared by a one-shot process in which the liquid hydrocarbon diol of Formula II, the multifunctional isocyanate, and optional polyol are combined with or without a catalyst and are allowed to react at room temperature or elevated temperatures to form a thermoplastic polyurethane or a thermoset polyurethane with the thermoplastic or thermoset nature depending on the structure/functionality of the particular ingredients chosen. Such polyurethanes are useful for a variety of end uses including the coating of flexible substrates that are used for fabrics, synthetic leather, as well as other decorative and functional end uses. When prepared in solution form or dissolved in a solvent, the thermoplastic polyurethanes can be prepared in film form by casting the solution onto a release medium such as release paper and removing the solvent by drying.

Illustrative of other thermoplastic and thermoset polyurethanes include those represented by the following formulae:

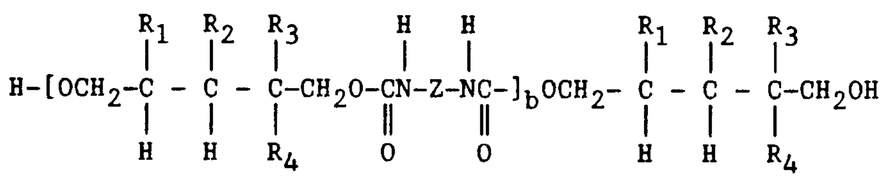

wherein $R_1$, $R_2$, $R_3$, $R_4$, and Z are as defined above, and b has a value of less than about 25 to more than about 1000;

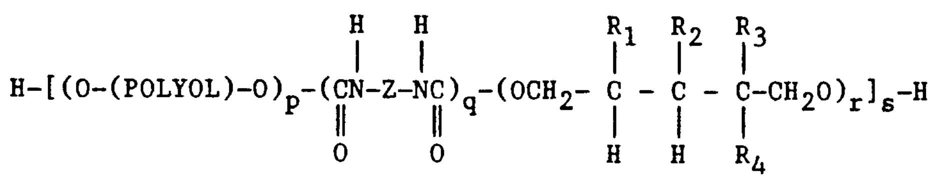

wherein $R_1$, $R_2$, $R_3$, $R_4$, Z and POLYOL are as defined above, p has a value of 1, q has a value of from about 2 to about 10, r has a value of from about 1 to about 9, and s has a value of from about 5 to about 500 with the most desirable values of s being dependent on the nature, molecular weight, and functionality of POLYOL; and the like, with it understood that when difunctional polyols and isocyanates are used thermoplastic products will result and when polyols and/or isocyanates of functionality greater than 2 are used polyurethanes with thermoset characteristics will be result.

In an embodiment of this invention, hydroxyl-terminated prepolymers, in which a ratio of two liquid hydrocarbon diol molecules are reacted with one diisocyanate molecule, are reacted with aminoplast resins; illustrative of which are the alkoxymelamines, alkylated benzoquanamines, methylated melamines, butylated melamines, butylated ureas, quanidienes, and the like; in the presence of strong acid catalysts; illustrative of which are para-toluene sulfonic acid, triflic acid, sulfonium salts, and the like; or are reacted with cycloaliphatic epoxides; illustrative of which are 3,4-epoxycyclohexylmethyl 3,4-epoxycyclohexane-carboxylate, 6-methyl-3,4-epoxycyclohexylmethyl 6-methyl-3,4-epoxycyclohexanecarboxylate, bis-(3,4-epoxycyclohexylmethyl) adipate, the compounds described in U.S. Patent No. 2,890,194, U.S. Patent No. 2,750,395, U.S. Patent No. 3,318,822, limonene diepoxide and the like; in the presence of triflic acid salts such as ammonium triflate, diethylammonium triflate, diisopropylammonium triflate, sulfonium salts, boron trifluoride salts, and the like.

Although it is not required, it is usually preferred that an inert solvent be used when the reactive polyurethanes of this invention are prepared. Illustrative of such solvents are toluene, methyl ethyl ketone, methyl amyl ketone, methyl i-butyl ketone, xylene, ethoxyethyl acetate, ethoxybutyl acetate, propoxyethyl acetate, dimethylformamide, ethyl acetate, butyl acetate and the like.

Polyurethane foams can be produced from the Formula II diols described above and polyfunctional isocyanates and optionally other polyols which are preferentially of tri- or higher functionality. If the Formula II diols are used alone to prepare the foams, a trifunctional or higher isocyanate will preferentially

be used. Foam formulations are well known to those skilled in the art of polyurethane foam manufacture and, in addition to the Formula II diols, such foams are prepared from di− or higher functionality isocyanates, trifunctional polyols, blowing agents, catalysts, surfactants, and other miscellaneous additives as described for example in J. H. Saunders and K. C. Frisch, "Polyurethanes, Chemistry and Technology: Part II. Technology," pp 4−7 (1964) which is incorporated herein by reference. Flexible foams are prepared by either a prepolymer technique, a semi− or quasi−prepolymer technique, or a one−shot method, all of which are described in J. H. Saunders and K. C. Frisch, "Polyurethanes, Chemistry and Technology: Part II. Technology," pp 7−50 (1964) which is incorporated herein by reference. In addition to the Formula II diols, other raw materials that may be used in the preparation of rigid foams are well known to those skilled in the art and are described for example in J. H. Saunders and K. C. Frisch, "Polyurethanes, Chemistry and Technology: Part II. Technology," pp 197−224 (1964) which is incorporated herein by reference. In the case of foams, the blowing agent and surfactant are required items in a formulation unless frothed foams are involved. In this latter instance, a gas such as dry nitrogen or air is whipped or beaten into the liquid ingredients causing a froth to form which is stabilized and reacted to form the foam.

The polyurethane foams of this invention can be used in a variety of end uses as described above, but a particular feature of the foam involves significant oil absorption when significant quantities of the Formula II diols are used. Such oil absorption allows the foam to be of eminent use in cleaning up oil spills as can occur when accidents happen during the various means of conveying crude or other oil products. In addition to high absorption, the oil is easily recovered by conveying the foam/oil system through rollers after which the foam can be reused to absorb more oil. Although this property of oil retention will be enhanced when any level of the Formula II diols are used, it is preferred that 25 percent or more of the Formula II diol be used in the foam manufacture.

In the manufacture of the polyurethanes of this invention, a variety of additives known to those skilled in the art can be used. Illustrative of such additives are pigments, fillers, and colorants; vinyl chloride/vinyl acetate copolymers, phenoxy polymers; silicones, silicone/alkylene oxide copolymers, and fluorochemical surfactants as well as other antiblocking, slip, and flow and leveling agents; and the like. These ingredients can be employed in conventional amounts known in the art.

As used herein, the term "polyol" is contemplated to include all permissible hydrocarbon compounds having 2 or more hydroxyl groups, e.g., diols, triols and the like.

For purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds which can be substituted or unsubstituted.

As used herein, the term "substituted" is contemplated to include all permissible substituents of organic compounds unless otherwise indicated. In a broad aspect, the permissible substituents include acyclic and cyclic, branched and unbranched, carbocyclic and heterocyclic, aromatic and nonaromatic substituents of organic compounds. Illustrative substituents include, for example, alkyl, alkyloxy, aryl, aryloxy, hydroxy, hydroxyalkyl, amino, aminoalkyl, halogen and the like in which the number of carbons can range from 1 to about 20 or more, preferably from 1 to about 12. The permissible substituents can be one or more and the same or different for appropriate organic compounds. This invention is not intended to be limited in any manner by the permissible substituents of organic compounds.

Certain of the following examples are provided to further illustrate this invention.

Glossary of Terms

Double Rubs − Solvent resistance was measured as the number of solvent (methyl ethyl ketone or acetone) double rubs that were required to cut through the coating. If 100 rubs or more did not cut through the coating, the coating was recorded as >100. To perform the test, the solvent−soaked cloth was rubbed back and forth with hand pressure. A rub back and forth was designated as one "double rub."

Pencil Hardness − Procedure conducted in accordance with ASTM D 3363−74.

Crosshatch Adhesion − Procedure conducted in accordance with ASTM D 3359−87.

Resin 1 − Resin used as one package of a two−package coating system and sold as Loctite 361 Resin by Loctite Corporation. MSDS indicates composition is 55−60 weight percent 1,1,1−trichloroethane, 20−25 weight percent polyisocyanate prepolymer, 15−20 weight percent 2−ethoxyethyl acetate, 5−7 weight percent methylene bisphenylisocyanate, 0.1−1 weight percent tert−butyl alcohol, and 0.1−1 weight percent methylal.

Activator 1 − Activator used as one package of a two−package coating system and sold as Loctite 361 Activator by Loctite Corporation. MSDS indicates composition is 50−55 weight percent high boiling

methacrylate, 25 – 30 weight percent polyurethane methacrylate resin, 5 – 7 weight percent acrylic acid, 3 – 5 weight percent hydroxyalkyl methacrylate, 3 – 5 weight percent photoinitiator (CAS #947 – 19 – 3), and 1 – 3 weight percent substituted silane.

Polyol I – A difunctional poly – $\epsilon$ – caprolactone polyol with a number average molecular weight of 540 and commercially available from Union Carbide Chemicals and Plastics Company Inc. as TONE® – 0200.

Melamine I – A polymethoxymethyl melamine commercially available from Monsanto Company as CYMEL® 303.

Catalyst I – An amine catalyst diluted with a glycol and commercially available from Union Carbide Chemicals and Plastics Company Inc. as A – 33.

Catalyst II – An amine catalyst diluted with a glycol and commercially available from Union Carbide Chemicals and Plastics Company Inc. as C – 174.

Catalyst III – A 55 percent solution of dinonylnaphthalene disulfonic acid in iso – butanol.

Surfactant I – A silicone/alkylene oxide surfactant commercially available from Union Carbide Chemicals and Plastics Company Inc. as SILVET® L – 5309.

Example 1

Isophorone diisocyanate, 75 grams (0.345 moles), and 100 grams of toluene were charged to a 500 milliliter, 4 – neck flask equipped with a mechanical stirrer, nitrogen sparger, inlet and outlet nitrogen connection which also functioned as a feeding port, and thermometer. While sparging with nitrogen and stirring, these ingredients were heated to 80 °C and held at this temperature to remove water. The temperature was then decreased to 55 °C and 30 grams (0.170 moles) of 2 – ethyl – 3 – propyl – 1,5 – pentanediol were slowly added to the reaction mass. The reaction was allowed to proceed at 55 °C for three hours, and then the reaction mass was allowed to cool to room temperature and stand overnight under a nitrogen blanket. A 20 gram portion of the reaction mass, which was a moisture – curable polyurethane, was removed and stored.

Example 2

A portion of the Example 1 moisture – curable polyurethane was applied to a phosphatized steel panel with a No. 20 wire – wound rod. The coating was cured by keeping it under ambient conditions so the toluene would evaporate and moisture present in the atmosphere could cure the product. After five days the coating was examined and found to be smooth, tack free and hard and to have high gloss. The coating had a 2B pencil hardness and 50 percent crosshatch adhesion. As was expected for the soluble, linear polymeric coating made, acetone resistance was poor and the coating could be cut through after 2 double acetone rubs. The coated panel was immersed in room temperature water for 24 hours and when tested on removal from the water, gloss remained unchanged by visual observation, and hardness and adhesion remained the same indicating that the aqueous environment did not soften, solubilize or otherwise have a deleterious effect on the coating.

Example 3

An 80/20 mixture of 2,4 – /2,6 – toluene diisocyanate, 60 grams (0.343 moles), and 100 grams of toluene were charged to a 500 milliliter, 4 – neck flask equipped with a mechanical stirrer, nitrogen sparger, inlet and outlet nitrogen connection which also functioned as a feeding port, and thermometer. While sparging with nitrogen and stirring, these ingredients were heated to 80 °C and held at this temperature to remove water. The temperature was then decreased to 55 °C and 30 grams (0.170 moles) of 2 – ethyl – 3 – propyl – 1,5 – pentanediol were slowly added to the reaction mass. The reaction was allowed to proceed at 55 °C for three hours, and then the reaction mass was allowed to cool to room temperature and stand overnight under a nitrogen blanket. A 20 gram portion of the reaction mass, which was a moisture – curable polyurethane, was removed and stored.

Example 4

A portion of the Example 3 moisture – curable polyurethane was applied to a phosphatized steel panel with a No. 20 wire – wound rod. The coating cured by keeping it under ambient conditions so the toluene would evaporate and moisture could cure the product. After standing overnight (approximately 16 hours), the coating was examined and found to be smooth, tack free and hard and to have high gloss. It had a B

pencil hardness and 100 percent crosshatch adhesion. As was expected for the soluble, linear polymeric coating made, acetone resistance was poor and the coating could be cut through after 2 double acetone rubs. After standing under ambient conditions for 20 days, the coating had an H pencil hardness and 100 percent crosshatch adhesion.

Examples 5 and 6 and Control Example A

These examples demonstrate that the moisture-curable coatings of this invention can be combined with ultraviolet light curable coatings and still cure over a period of time due to moisture in the atmosphere. The ingredients listed in Table A below were placed in amber-colored bottles and mixed well for each example. They were then applied to phosphatized steel panels and exposed to a 300 watt-per-inch medium pressure mercury vapor lamp at 30 feet per minute. The coatings were tested by the indicated tests in Table A and the results are given in Table A.

Table A

| Ingredients, grams | Example 5 | Example 6 | Control A |
|---|---|---|---|
| Resin 1 | 5.0 | 5.0 | 5.0 |
| Activator 1 | 10.0 | 10.0 | 10.0 |
| Example 3 Polyurethane | 1.5 | 3.0 | --- |
| Properties 1 day after Ultraviolet Light Exposure | | | |
| Acetone Double Rubs | 10(3) | 8(3) | 20(3) |
| Pencil Hardness | <6B | <6B | 3B |
| Crosshatch Adhesion, % | 100 | 100 | 100 |
| Properties 20 days after Ultraviolet Light Exposure | | | |
| Acetone Double Rubs | 15(3) | 14(3) | 20(3) |
| Pencil Hardness | 4B | 3B | 3B |
| Crosshatch Adhesion, % | 100 | 100 | 100 |

Example 7

A moisture-curable polyurethane was prepared from mixture of dihydroxyl-functional and trihydroxyl-functional compounds and a difunctional isocyanate. In the same manner as described in Example 3, 32.54 grams (0.3740 equivalents) of an 80/20 mixture of 2,4-/2,6-toluene diisocyanate and 100 grams of toluene were charged to a 500 milliliter, 4-neck flask equipped with a mechanical stirrer, nitrogen sparger, inlet and outlet nitrogen connection which also functioned as a feeding port, and thermometer. While sparging with nitrogen and stirring, these ingredients were heated to 80°C and held at this temperature to remove water and dry the diisocyanate. The temperature was then decreased to 55°C and 27 grams (0.3068 equivalents) of 2-ethyl-3-propyl-1,5-pentanediol and 3.0 grams (0.0672 equivalents) of trimethylolpropane were slowly added to the reaction mass over a 30 to 40 minute period. The reaction was allowed to proceed at 55°C for three hours, and then the reaction mass was allowed to cool to room temperature and stand overnight under a nitrogen blanket during which the moisture-curable polyurethane product separated from the toluene.

The product was soluble in both acetone and methyl isobutyl ketone. A small amount of the product was dissolved in these solvents and coated onto phosphatized steel panels with a number 20 wire wound rod. After approximately both 30 minutes and 1.5 hours under ambient conditions, the coating from acetone was tack free and was cut through by two double acetone rubs. After 18 hours under these conditions the coating was dull in appearance and was marred after two acetone double rubs, had a 2H pencil hardness, and had 100 percent crosshatch adhesion. After three hours under ambient conditions, the coating from methyl isobutyl ketone had high gloss, a pencil hardness of 2H, and 100 percent crosshatch adhesion. After standing 12 days under ambient conditions, the properties had improved to a pencil hardness of >6H and crosshatch adhesion was 100 percent.

Example 8

A thermoplastic polyurethane coating solution is prepared by placing 2.0 equivalents of 2-ethyl-3-propyl-1,5-pentanediol and one equivalent of an approximately 800 molecular weight poly-$\epsilon$-caprolactone diol in a glass reaction flask that is equipped with a stirrer, thermometer, nitrogen sparging device, and an inlet port. The compounds are heated to 110°C and held at that temperature while stirring and sparging with dry nitrogen to remove water. The temperature is then decreased to 40°C and sufficient methyl isobutyl ketone is added so that there is 70 percent of this solvent in the final mixture. Then, 0.02 percent by weight, based on the total weight of reactive ingredients used, of dibutyltin dilaurate is added. With the temperature at 40°C, three equivalents of an 80/20 mixture of 2,4- and 2,6-toluene diisocyanate are added over a 10- to 15-minute period. There will be an exotherm taking place during addition of the diisocyanate, and it is controlled by cooling. The reaction mixture is maintained at 40-50°C for three hours after which the reaction mass is cooled to room temperature.

Example 9

A thermoplastic polyurethane coating solution is prepared by placing 1.95 equivalents of 2-ethyl-3-propyl-1,5-pentanediol in a glass reaction flask that is equipped with a stirrer, thermometer, nitrogen sparging device, and an inlet port. The diol is heated to 45°C and held at that temperature while stirring and sparging with dry nitrogen. The temperature is then decreased to about 40°C and sufficient solvent, which is a 2/1 blend of methyl ethyl ketone and dimethylformamide, is added so that there is 70 percent of this solvent in the final mixture. With the temperature at about 40°C, 0.02 percent by weight, based on the total weight of reactive ingredients used, of dibutyltin dilaurate is added. Then three equivalents of 4,4'-dicyclohexylmethane diisocyanate are added over a 10- to 15-minute period. If an exotherm takes place during addition of the diisocyanate, it is controlled by cooling. The reaction mixture is maintained at 40-50°C for six hours after which the reaction mass is cooled to room temperature and allowed to remain in the reaction flask under a nitrogen blanket over night. The mixture is then heated to about 70°C and 1.0 equivalent of a dry 1,000 molecular weight poly(tetramethylene oxide) diol and 0.05 equivalent of dry trimethylolpropane are added. The reaction is allowed to continue at about 70°C for five hours after which time the system is cooled to room temperature.

Example 10

A polyurethane foam is prepared by blending 4.35 grams of 2-ethyl-3-propyl-1,5-pentanediol, 3.0 grams of water, 1.0 grams of glycerine, 0.6 grams of Catalyst I, 0.4 grams of Catalyst II, and 0.5 parts of Surfactant I. This mixture is placed in a container, and 46 grams of an 80/20 mixture of 2,4-/2,6-toluene diisocyanate are added. The components are rapidly mixed with mechanical agitation for about 2 to 10 seconds, the mixer is removed, and a cold-cure foam forms.

Example 11

A second polyurethane foam is prepared in the same manner as described in Example 10 except 50 grams of a trifunctional propylene oxide polyol with an equivalent weight of 2,000 and 2.17 grams of 2-ethyl-3-propyl-1,5-pentanediol are substituted for the 4.35 grams of 2-ethyl-3-propyl-1,5-pentanediol used in Example 10.

Examples 12 and 13

These examples demonstrate that thermoplastic polyurethanes can be made from the hydrocarbon diol and combinations of hydrocarbon diols and other polyols. Thermoplastic polyurethanes were prepared from the ingredients listed in Table B below in a 250 milliliter glass reaction flask equipped with a stirrer, thermometer, gas inlet and outlet, and feeding port. Toluene and 2-ethyl-3-propyl-1,5-pentanediol were placed in the reaction flask and heated to 100°C and held at this temperature for 30 minutes while sparging with dry nitrogen to remove any water that was in the ingredients. The temperature was then decreased to 55°C and the toluene diisocyanate was slowly added over a 15 to 20-minute period. After the addition was complete, the reaction temperature was maintained at 55°C for 3 hours after which time the methoxyhydroquinone was added. The product, which was of low viscosity and clear, was then stored for future use. Coatings were prepared on phosphatized steel with a No. 32 wire-wound rod and allowed to air dry overnight, about 14 hours, under ambient conditions. The clear, thermoplastic coatings had the properties indicated in Table B.

Table B

| Ingredients, grams/equivalents | Examples 12 | Examples 13 |
|---|---|---|
| Toluene | 80.0 | 160.0 |
| 2-Ethyl-3-propyl-1,5-pentanediol | 4.33/0.050 | 10.0/0.115 |
| Polyol I | ---- | 4.5/0.017 |
| Toluene diisocyanate* | 4.35/0.050 | 5.8/0.067 |
| Properties on phosphatized steel | | |
| Pencil Hardness | H | 2B |
| Percent Crosshatch Adhesion | 100 | 100 |

*An 80/20 mixture of 2,4- and 2,6-toluene diisocyanate.

Examples 14 and 15

The ingredients listed in Table C below were reacted in a 3-necked glass reaction flask equipped with a stirrer, thermometer and dry nitrogen inlet and outlet. The 2-ethyl-3-propyl-1,5-pentanediol and trimethylolpropane were placed in the reaction flask and heated to 100°C with nitrogen sparging and held under these conditions for one hour. The temperature was then decreased to 65°C and the isophorone diisocyanate was added slowly. A 5°C exotherm was noticed during the addition of isophorone diisocycanate. Stannous octanoate was then added and the temperature was maintained at 65°C for one hour. After this time period, the toluene was added and the reaction continued for an additional 2.5 hours. The resulting clear, low viscosity hyroxyl-terminated products were cooled to room temperature and stored for future use.

Table C

| Ingredients, grams | Example 14 | Example 15 |
|---|---|---|
| 2-Ethyl-3-propyl-1,5, pentanediol | 18.0 | 18.0 |
| Trimethylolpropane | 2.0 | 2.0 |
| Isophorone Diisocyanate | 14.0 | 21.5 |
| Stannous Octanoate | 0.017 | 0.017 |
| Toluene | 10.0 | 20.0 |

Examples 16 and 17

These examples describe the preparation of thermally curable coatings prepared from the polyurethanes of Examples 14 and 15, an aminoplast cross linking agent and other ingredients listed in Table D below. After thorough mixing of the ingredients, the resulting mixtures were coated onto phosphatized steel panels with a No. 20 wire-wound rod and cured at 140°C for 30 minutes. Hard clear coatings resulted. The coatings were then tested by the indicated tests in Table D and the results are given in Table D.

Table D

| Ingredients, grams | Example 16 | Example 17 |
|---|---|---|
| Example 14 Polyurethane | 9.06 | -- |
| Example 15 Polyurethane | -- | 12.6 |
| Melamine I | 3.0 | 3.0 |
| Catalyst III | 0.05 | 0.05 |
| Surfactant I | 0.12 | 0.12 |
| Surfactant II | 0.12 | 0.12 |
| Methyl Amyl Ketone | 2.50 | 2.50 |
| Cured Film Properties | | |
| Cured film thickness, mils | 2.2 | 2.0 |
| Specular Gloss (20°) (ASTM D 523-78) | 80.6 | 61.6 |
| Specular Gloss (60°) (ASTM D 523-78) | 98.3 | 92.2 |
| Pencil Hardness (ASTM D 336-3-74) | 2H | 2H |
| Impact Resistance (ASTM D 2794-84) | | |
| Forward, in-lbs. | 50 | 50 |
| Reverse, in-lbs. | 10 | 10 |
| Crosshatch Adhesion | 100 | 100 |

Although the invention has been illustrated by certain of the preceding examples, it is not to be construed as being limited thereby; but rather, the invention encompasses the generic area as hereinbefore disclosed. Various modifications and embodiments can be made without departing from the spirit and scope thereof.

Related Applications

The following are related, commonly assigned applications, filed on an even date herewith:

U.S. Patent Application Serial No. 790,875 (D−16840); U.S. Patent Application Serial No. 790,872 (D−16841); U.S. Patent Application Serial No. 790, 790,873 (D−16842); U.S. Patent Application Serial No. 790,874 (D−16844); and U.S. Patent Application Serial No. 790,895 (D−16845); all of which are incorporated herein by reference.

**Claims**

**1.** A polyurethane comprising the reaction product of (i) a liquid hydrocarbon diol comprised of primary hydroxyl groups and 8 or more carbon atoms in which the primary hydroxyl groups are separated by 4 or more carbon atoms linearly arranged and in which at least one of said carbon atoms linearly arranged is a disubstituted carbon atom or at least 2 of said carbon atoms linearly arranged are monosubstituted carbon atoms, said liquid hydrocarbon diol existing as a liquid at a temperature of 35 ° C or less, and/or a derivative of said liquid hydrocarbon diol, and (ii) a polyfunctional isocyanate.

**2.** The polyurethane of claim 1 further comprising a polyol different from said liquid hydrocarbon diol or derivative thereof.

**3.** The polyurethane of claim 1 wherein the liquid hydrocarbon diol is represented by one of the following formulae:

HO − R' − OH (I)

wherein R' is a substituted hydrocarbon residue having 8 or more carbon atoms in which the primary hydroxyl groups are separated by 4 or more carbon atoms linearly arranged and in which at least one of said carbon atoms linearly arranged is a disubstituted carbon atom or at least 2 of said carbon atoms linearly arranged are monosubstituted carbon atoms; or

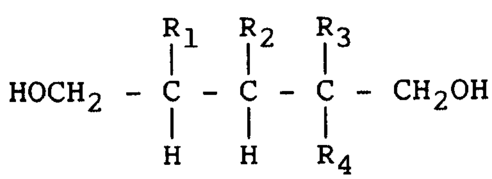

(II)

wherein $R_1$ is hydrogen or linear or branched alkyl having from 1 to 3 carbon atoms, and $R_2$, $R_3$ and $R_4$ are the same or different and are hydrogen or linear or branched alkyl having from 1 to 4 carbon atoms; or

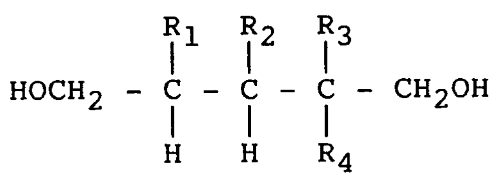

(III)

wherein $R_1$ is hydrogen or linear or branched alkyl having from 1 to 3 carbon atoms, and $R_2$, $R_3$ and $R_4$ are the same or different and are hydrogen or linear or branched alkyl having from 1 to 4 carbon atoms; provided (a) at least 2 of $R_1$, $R_2$, $R_3$ and $R_4$ are other than hydrogen; (b) the total number of carbon atoms in $R_1$, $R_2$, $R_3$ and $R_4$ together is 5 or more except as follows: (i) when $R_2$ is ethyl and one of $R_1$, $R_3$ and $R_4$ is methyl, then the remaining 2 of $R_1$, $R_3$ and $R_4$ can be hydrogen; (ii) when $R_3$

and $R_4$ are independently methyl and propyl, then $R_1$ and $R_2$ can be hydrogen; and (iii) when $R_1$ is methyl and $R_2$ is ethyl and one of $R_3$ and $R_4$ is methyl, then the remaining $R_3$ or $R_4$ can be hydrogen; (c) when the total number of carbon atoms in $R_1$ and $R_2$ is 4 or more, then the total number of carbon atoms in $R_2$ is 3 or less except when $R_1$ is propyl and $R_3$ and $R_4$ are hydrogen, then $R_2$ can be butyl; and (d) when $R_1$ is ethyl and $R_2$ is propyl, then $R_3$ is other than ethyl.

**4.** The polyurethane of claim 1 in which the liquid hydrocarbon diol is selected from 3-ethyl-2-methyl-1,5-pentanediol, 2-ethyl-3-propyl-1,5-pentanediol, 2,4-dimethyl-3-ethyl-1,5-pentanediol, 2-ethyl-4-methyl-3-propyl-1,5-pentanediol, 2,3-diethyl-4-methyl-1,5-pentanediol, 3-ethyl-2,2,4-trimethyl-1,5-pentanediol, 2,2-dimethyl-4-ethyl-3-propyl-1,5-pentanediol, 2-methyl-2-propyl-1,5-pentanediol, 2,4-dimethyl-3-ethyl-2-propyl-1,5-pentanediol, 2,3-dipropyl-4-ethyl-2-methyl-1,5-pentanediol, 2-butyl-2-ethyl-1,5-pentanediol, 2-butyl-2,3-diethyl-4-methyl-1,5-pentanediol, 2-butyl-2,4-diethyl-3-propyl-1,5-pentanediol, 3-butyl-2-propyl-1,5-pentanediol, and mixtures thereof, and the polyfunctional isocyanate is selected from 2,4-toluene diisocyanate, 2,6-toluene diisocyanate, 4,4'-diphenylmethane diisocyanate (MDI), 4,4'-dicyclohexyldiisocyanate, meta- and para-tetramethyl xylene diisocyanate, 3-isocyanatomethyl-3,5,5,-trimethylcyclohexylisocyanate, hexamethylene diisocyanate, 2,2,4- and 2,4,4-trimethylenehexamethylene diisocyanate, meta-and para-phenylene diisocyanate, isophorone diisocyanate, 2,4-, 2,6- and 2,4-,2,6-bromotoluene diisocyanate, 4-bromo-meta-phenylene diisocyanate, ortho- and meta-trifluoromethylphenylisocyanate; ortho, meta-, and para-fluorophenylisocyanate; 4,6-dibromo-meta-phenylene diisocyanate; ortho-, meta-, and para-chlorophenyl isocyanate, 4,4',4''-triisocyanatotriphenylmethane, and mixtures thereof.

**5.** The polyurethane of claim 1 in which the polyfunctional isocyanate is represented by the formula:

$Z-(NCO)_n$

wherein Z is a substituted or unsubstituted hydrocarbon residue and n is a value of from 2 to about 6.

**6.** The polyurethane of claim 1 represented by the formula:

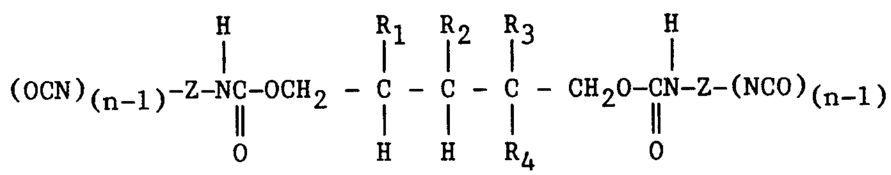

wherein $R_1$ is hydrogen or linear or branched alkyl having from 1 to 3 carbon atoms, $R_2$, $R_3$ and $R_4$ are the same or different and are hydrogen or linear or branched alkyl having from 1 to 4 carbon atoms, Z is the same or different and is a substituted or unsubstituted hydrocarbon residue, and n is a value of from about 2 to about 6.

**7.** A process for preparing a polyurethane which comprises contacting a liquid hydrocarbon diol comprised of primary hydroxyl groups and 8 or more carbon atoms in which the primary hydroxyl groups are separated by 4 or more carbon atoms linearly arranged and in which at least one of said carbon atoms linearly arranged is a disubstituted carbon atom or at least 2 of said carbon atoms linearly arranged are monosubstituted carbon atoms, said liquid hydrocarbon diol existing as a liquid at a temperature of 35°C or less, and/or a derivative of said liquid hydrocarbon diol, with a polyfunctional isocyanate to form said polyurethane.

**8.** A two-package polyurethane system comprising (i) a first package comprised of a polyurethane of claim 1, and (ii) a second package comprised of a polyol.

**9.** A thermoplastic or thermoset polyurethane represented by the formula:

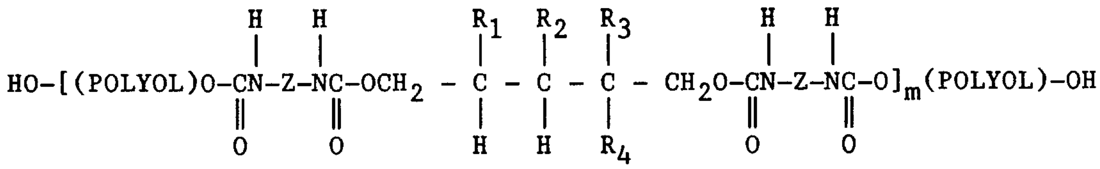

wherein $R_1$ is the same or different and is hydrogen or linear or branched alkyl having from 1 to 3 carbon atoms, $R_2$, $R_3$ and $R_4$ are the same or different and are hydrogen or linear or branched alkyl having from 1 to 4 carbon atoms, Z is the same or different and is a substituted or unsubstituted hydrocarbon residue, POLYOL is the same or different and is a substituted or unsubstituted hydrocarbon residue, and m has a value of from 25 or less to about 150 or greater.

**10.** A curable coating composition comprising the polyurethane of claim 1 or a cured film prepared therefrom, or an adhesive, ink or sealant composition comprising the polyurethane of claim 1, or a polyurethane foam comprised of the polyurethane of claim 1, a blowing agent and a surfactant.

European Patent Office

# EUROPEAN SEARCH REPORT

Application Number

EP 92 11 9270

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 740 639 (W.A. BEAVERS)<br>* column 1, line 14 - line 16 *<br>* column 2, line 19 - line 21 *<br>* column 2, line 59 - column 3, line 6 *<br>* column 4, line 53 - line 56 *<br>* column 4, line 66 - column 5, line 4 *<br>--- | 1-5 | C08G18/32<br>C08G18/80<br>C08G18/65<br>C09D175/04<br>C09J175/04<br>C09K3/10<br>C09D11/00<br>C07C271/20<br>C07C271/24<br>C07C271/28<br>//(C08G18/32, 101:00) |
| A | FR-A-2 284 160 (BASF)<br>* claim 1 *<br>* page 4, line 20 - page 5, line 9 *<br>----- | 1 | |
| | | | TECHNICAL FIELDS SEARCHED (Int. Cl.5) |
| | | | C08G<br>C07C |

The present search report has been drawn up for all claims

| Place of search | Date of completion of the search | Examiner |
|---|---|---|
| THE HAGUE | 02 MARCH 1993 | VAN PUYMBROECK M. A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document